Europäisches Patentamt

European Patent Office

Office européen des brevets

(19)

(11) Publication number: **0 015 082**
**B1**

(12) **EUROPEAN PATENT SPECIFICATION**

(45) Date of publication of patent specification: **25.05.83**

(21) Application number: **80300287.2**

(22) Date of filing: **31.01.80**

(51) Int. Cl.³: **C 12 N 1/00, C 12 N 1/32,
A 23 K 1/00**

(54) **Single cell protein and its production.**

(30) Priority: **27.02.79 GB 7906829**

(43) Date of publication of application:
**03.09.80 Bulletin 80/18**

(45) Publication of the grant of the patent:
**25.05.83 Bulletin 83/21**

(84) Designated Contracting States:
**AT BE CH DE FR GB IT LU NL SE**

(56) References cited:
**CH - A - 508 040
FR - A - 343 712
FR - A - 2 110 156
GB - A - 1 207 473
US - A - 3 904 476
US - A - 4 154 726**

**CHEMICAL ABSTRACTS, vol. 90, no. 9, 26th
February 1979, page 393, no. 70617p
Columbus, Ohio, U.S.A.
CHEMICAL ABSTRACTS, vol. 75, no. 25, 20th
December 1971, page 207, no. 150329b
Columbus, Ohio, U.S.A.**

(73) Proprietor: **IMPERIAL CHEMICAL INDUSTRIES
PLC
Imperial Chemical House Millbank
London SW1P 3JF (GB)**

(72) Inventor: **Talbot, Colin John
2 Common Road
Eton Wick, Windsor Berkshire (GB)**
Inventor: **Senior, Peter James
Foulis Cottage Ingleby Greenhow
Middlesbrough, Cleveland (GB)**

(74) Representative: **Aufflick, James Neil et al,
Imperial Chemical Industries PLC Legal
Department: Patents Thames House North
Millbank
London SW1P 4QG (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Single cell protein and its production

This invention relates to an improved process for the production of single cell protein (SCP).

Proteinaceous materials are important dietary components for many animals. A wide variety of such materials are in use including for example fish meal, soya bean meal and materials derived from rape seeds and sunflower seeds. In the past twenty years considerable research effort has been devoted to the production of yeast and bacterial proteins for inclusion in animal diets. Such proteins derived from micro-organisms are commonly referred to as single cell protein or SCP.

The inclusion of high proportions of some widely used proteinaceous materials in their diets can have adverse effects upon certain species. This is frequently ascribed to "toxic" constituents. The effect of these constituents has been recently reviewed (Toxic Constituents of Plant Foodstuffs, Ed. I. E. Liener, Academic Press, New York). Liener (1969, pages 4—6) refers to toxic constituents as substances that produce deleterious effects when fed to men or animals. He distinguishes between chemical constituents that are lethal and those that produce an adverse physiological response. Processing, or "other means of preparation have proved to be effective in destroying many toxic constituents". Reference is also made to Altshul A. M., (1967, Science, *158*, 221) that if it "were not for the fact that such oilseeds as soyabeans and cotton seed can be processed so as to inactivate their toxic constituents, these rich sources of plant protein would not occupy such a position of importance as they now command in the feeding of farm animals".

It is known that the feeding of single cell protein derived from prokaryotic micro-organisms, which include bacteria and blue-green algae, to certain animal species, particularly domestic fowls, in high dietary inclusions causes reduced feed intake (RFI), reduced live weight gain (LWG) and poor feed conversion ratios (FCR). In extreme instances this produces liver damage with organ enlargement, focal necrosis with phagocytic and inflammatory cell infiltration being observable on histopathological examination. This effect has been produced with prokaryotic single cell protein derived from different micro-organisms, grown on different nutrient media and in different processes and has been reported by (i) D. E. Owen et al, (Fd. Cosmet. Toxicol, *16*, 633, 1978); (ii) L. Farstad (Acta Agric. Scand., *27*, 129, 1977); (iii) C. T. Whittemore et al, (British Poultry Science, *19*, 283, 1978) and (iv) personal information supplied by Dr. K. Kameoka, National Institute of Animal Husbandry, Chibi-shi, 280, Japan.

From experiments in which single cell protein produced by the process described in our GB—A—No. 1 370 892 was included as a proteinaceous material in normal commercial diets fed to domestic fowls, we have found progressive decreases in LWG and progressive decreases in food intake. Correlated with this is an increase in the incidence of observable liver damage as the percentage inclusion of prokaryotic single cell protein in the diets is increased. The experiments were carried out with diets containing 0%, 2.5%, 5%, 7.5%, 10%, 15%, 20%, 25%, and 30%, of prokaryotic single cell protein produced by the process of GB—A—No. 1 370 892. With low inclusions of SCP (e.g. 2.5%), the incidence of liver damage did not differ significantly from the incidence with diets containing no SCP. However, with high inclusions of SCP significant differences in the incidence of liver damage between SCP containing diets and non-SCP diets were observed. For instance with broiler chick diets containing non-stressful levels of crude protein, say 25%, and also non-stressful levels of vitamins and minerals, the incidence of liver damage was between 10 and 40% usually 10%. With laying hens, fed stressful levels (30%) of crude protein, contained in diets having a 30% SCP content the incidence of liver damage was between 30 and 70%, usually 50%. We have also found that the effect upon growth performance of domestic fowls resulting from the inclusion of SCP in their diets is not affected significantly by varying the microbial culture conditions or by varying the method whereby the microorganisms are dried to produce a dietary ingredient, i.e. freeze-, flash and spray-dried products give similar effects.

The effect of using prokaryotic SCP as a dietary ingredient upon certain species restricts the proportions in which the SCP can be included in the diets of these species and, in consequence, reduces the utility of prokaryotic SCP in relation to these species.

GB—A—1 207 473 discloses treatment of single cell protein with hydrogen peroxide at various pH values for the purpose of bleaching the single cell protein. However this reference does not disclose a treatment at a pH between 7.0 and 9.0 and does not consider the problem which the present invention has been developed to overcome.

According to the present invention we provide a process for the production of single cell protein which comprises cultivating prokaryotic microorganisms and subsequently treating the cultivated microorganisms to produce a product comprising whole non-viable cells and/or ruptured cells wherein the microorganisms are treated with an oxidising agent at a pH between 7.0 and 9.0, the oxidising agent being added during cultivation and/or during the subsequent treatment and/or to the product after the subsequent treatment, the oxidising agent being added in an amount between 3 and 300 m moles per litre of a solids suspension containing the microorganisms when it is added during cultivation or during subsequent treatment, or, when added to the product, being added in an amount between 3 and 300 m moles per litre of the product moisture content.

The prokaryotic microorganisms generally used to date in processes for the production of single

**0 015 082**

cell protein are bacteria. Thus the process of the present invention is mainly of interest insofar as it relates to bacteria and will hereafter be described as a process for the production of bacterial SCP. The present invention is applicable to any process for the production of bacterial SCP. Such a process is that described in our GB—A—No. 1 370 892 in which the bacteria employed are strains of the species *Pseudomonas methylotropha* (now known as *Methylophilus methylotrophus*), *Pseudomonas rosea*, *Hyphomicrobium variabile* and *Microcyclus polymorphum*. A number of strains of these species have been deposited at the following culture collections:—

(a)  National Collection of Industrial Bacteria (NCIB), Torrey Research Station, Aberdeen, Scotland, UK;
(b)  US Department of Agriculture (NRRL), Peoria, Illinois, USA;
(c)  Fermentation Research Institute (FRI), Japan;

and have been assigned the following accession numbers:—

NCIB Nos. 10508—10515; 10592—10596; 10516; 10517; 10597—10612.
NRRL Nos. B5352—B5359; B5360—B5364; B5381; B5382; B5365—B5380.
FRI Nos. 1215—1222; 1223—1227; 1228; 1229; 1230—1245.

The characteristics of the above four species and the particular strains listed are given in our GB—A—No. 1 370 892. Of the species specifically mentioned above *Pseudomonas methylotropha* (*Methylophilus methylotrophus*) is especially preferred. Among the specific strains listed above this species is represented by strains NCIB Nos. 10508—10515 and 10592—10596.

The process of the invention may be a batch or a continuous process preferably it is a continuous process. In a continuous process a culture of micro-organisms is grown in a fermenter and an aqueous suspension containing microbial cells is continuously withdrawn and subjected to a series of treatment steps to concentrate the suspension before it passes to a drier. In a typical process sequence the suspension leaving the fermenter is subjected to the following steps before passing to a drier:—

(i)  a flocculation step, e.g. involving heating and acid treatment as described in our GB—A—No. 1 381 306;
(ii)  a separation step or steps to separate the bulk of the liquid from the microbial cells, separated liquid may be returned to the fermenter while a concentrated suspension often termed "drier feed cream" passes to the drier.

The cells may be treated with the oxidising agent at any stage in the process. However in choosing the stage at which to perform treatment the following factors are relevant:

1.  The oxidising agent employed may be toxic to living micro-organisms and thus have deleterious effects upon a growing culture;
2.  Many oxidising agents are degraded and wholly or partially de-activated by enzymes such as peroxidases and catalase which are present in most bacteria;
3.  The amount of oxidising agent required depends upon the volume of liquid associated with the cells and thus the invention is more economically applied by adding the oxidising agents to concentrated suspensions or to the product;
4.  Treatment with oxidising agent is preferably carried out at temperatures between 10° and 140°C, especially between 50° and 80°C, a temperature of 60°C being particularly preferred;
5.  The pH at which treatment with oxidising agent is carried out is in the range pH 7.0—9.0, preferably in the range 7.5—8.5 with pH values of 8.0 to 8.5 being most suitable.

Taking account of the above considerations addition of the oxidising agent to a growing culture is not preferred because of problems caused by the action of the oxidising agent on a growing cell and by its degradation by catalase peroxidase. After cell lysis and suspension concentration catalase degradation is minimal and the solids concentration is high. Thus it is preferred to treat the cell suspension with the oxidising agent shortly before the suspension passes to the drier, i.e. to treat the drier feed cream. Alternatively the product from the drier may be treated with the oxidising agent. Although this product tends to be referred to loosely as "dried" cells, there is in fact a measurable proportion of water associated with the "dried" cells typically 10% (w/w). Thus if the oxidising agent is added in an amount of 3 to 300 m moles per litre of the product moisture content, this can be calculated as follows:

Typical "dry" product contains 10% moisture=100 mls water/kg.
Therefore add 3—300 m moles oxidising agent per 10 kg product.

When the product is treated with certain oxidising agents further process steps may be required in order to remove traces of the oxidising agents from the final product.

3

Suitably the oxidising agent is one which is pure, leaves no toxic residues and does zero or minimal damage to the nutritional quality of the product. The preferred oxidising agent is hydrogen peroxide. Other suitable oxidising agents include organic peroxides, inorganic peroxides, ozone and permanganate. Other suitable oxidising means may include electrolytic processes.

The preferred amount of oxidising agent to be added varies to some extent depending upon the pH at which addition takes place. However a preferred range of amounts effective at a variety of pH values is from 30 to 175 m moles, particularly 70 to 150 m moles, per litre of a solids suspension or per litre of the product moisture content. In the studies we have made we have found the greatest effect with minimal oxidation when adding the oxidising agent in an amount of 86 m moles per litre.

The duration of the treatment with the oxidising agent is suitably within the range 1 to 300 minutes, 10 to 40 minutes and particularly 30 minutes being preferred.

Using the process of the invention a product is obtained which has a number of advantages over previously known prokaryotic SCP. Most importantly use of this product alleviates the difficulties of reduced feed intake, reduced live weight gain and poor feed conversion ratios experienced with domestic fowls and reduces the incidence of liver damage when the product is fed at high dietary inclusions. In addition the product has improved colour and improved dispersion properties which are important when it is used for feeding young animals, e.g. veal calves. These advantages increase the utility of prokaryotic SCP generally.

The invention is illustrated by the following Examples:—

Example 1

In a series of large-scale trials male and female chicks and laying hens were fed diets containing *Methylophilus methylotrophus (Pseudomonas methylotropha)* single cell protein in some instances untreated and in others treated with hydrogen peroxide by the process of the present invention.

During production of the treated single cell protein used, cell lysate (i.e. drier feed cream) having a solids content of 18% (w/v) was treated with quantities of concentrated hydrogen peroxide solution (12% w/v) such that the initial concentration of hydrogen peroxide in the cream was between zero and 175 mM. After mixing the hydrogen peroxide with the cream, the treated cream was held at a temperature of 60°C and at pH 7.0 for 30 minutes prior to drying by flash drier.

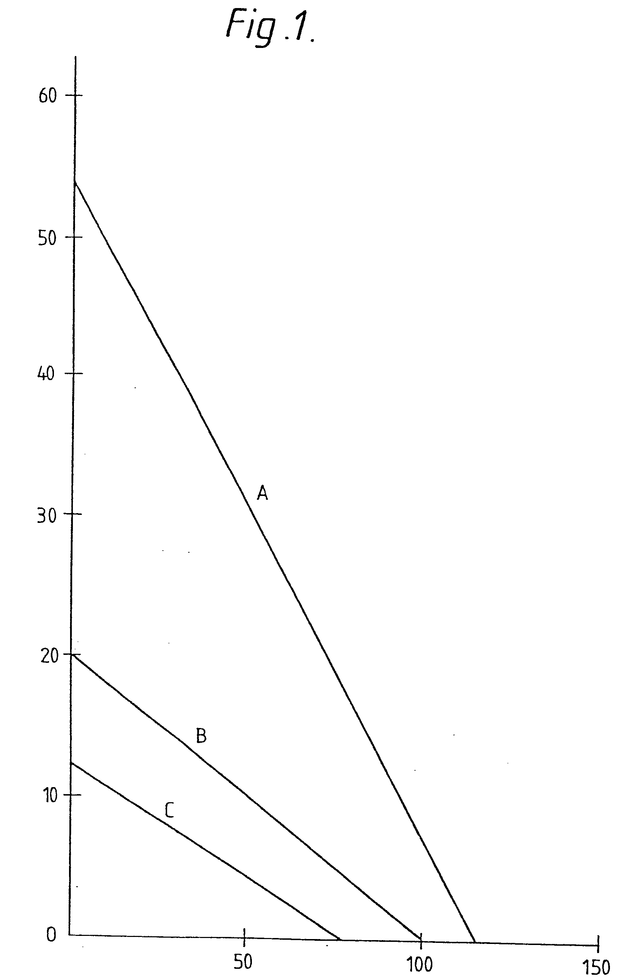

Typical results, for birds fed diets containing treated and untreated single cell protein and for birds fed diets in which single cell protein was replaced by alternative proteinaceous materials, are given in Table 1. Figure 1 is a graph of the occurrence of gross observable liver damage (per cent of the test population) against the concentration (mM)* of hydrogen peroxide in the drier feed cream for laying hens (plot A), female chicks (plot B) and male chicks (plot C). Plots A, B and C are linear regression analyses of all chick and laying hen data obtained in this series of trials. The incidence of lesions was highest with laying hens and least with male chicks. The graph indicates that the minimum concentration of hydrogen peroxide required to reduce liver damage in chicks and laying hens effectively to zero levels is 114 mM. In our tests on birds fed diets containing alternative proteinaceous materials a number of birds exhibited liver damage.

TABLE 1

| single cell protein in diet (% w/w) | mM $H_2O_2$* in drier feed cream | liver damage in laying hens | | liver damage in male chicks | | liver damage in female chicks | |
|---|---|---|---|---|---|---|---|
| | | incidence i.e. % of birds developing damage | total no. of birds in trial | incidence i.e. % of birds developing damage | total no. of birds in trial | incidence i.e. % of birds developing damage | total no. of birds in trial |
| 0.0 | 0 | 1.3 | 78 | 0.7 | 1067 | — | — |
| 7.5 | 0 | — | — | 5.2 | 408 | — | — |
| 10.0 | 0 | 8.3 | 60 | — | — | — | — |
| 25.0 | 0 | — | — | 15.3 | 144 | 21.0 | 276 |
| 30.0 | 0 | 47.1 | 102 | — | — | — | — |
| 25.0 | 35.0 | — | — | 5.9 | 187 | 16.5 | 284 |
| 25.0 | 175.0 | — | — | 2.0 | 100 | 0.0 | 93 |
| 30.0 | 70.0 | 19 | 42 | — | — | — | — |
| 30.0 | 87.5 | 7.1 | 42 | — | — | — | — |

* In the Table and in Figure 1 the concentration of hydrogen peroxide in m moles per litre was calculated assuming the drier feed cream to have a density of 1.0 g/cm³.

Example 2

In a further series of large-scale trials domestic fowls were fed diets containing *Methylophilus*

*methylotrophus (Pseudomonas methylotropha)* SCP, in some instances untreated and in others treated with hydrogen peroxide according to the process of the present invention. The SCP was produced in a large pilot plant using the process of our GB—A—No. 1370892. In the process bacterial cells were cultivated in a methanol-containing medium in a fermenter of our GB—A—No. 1 353 008. The cell suspension leaving the fermenter was treated using the method of our GB—A—No. 1 381 306 and was concentrated to produce a drier feed cream (DFC) which was subsequently fed to a drier.

In these trials samples of DFC at pH 4 and having a 12—15% solids content were taken from the plant and were subjected to the following treatment steps in a laboratory to produce hydrogenperoxide-treated material (steps 2 to 4 being omitted to produce a non-hydrogen peroxide treated control material):—

1.   add KOH/NaOH (1.68:1.00 molar ratio) to give desired pH;
2.   add $H_2O_2$—see note below;
3.   maintain at 60°C for 30 minutes;
4.*  re-neutralize to pH 7 with $H_2SO_4$;
5.   spray dry; and
6.   granulate feed sample.

*    In trial No. APC021J this step was found to be unnecessary.

In step 3 an amount of 12% (40 vol) hydrogen peroxide was added to the DFC. The number of millilitres of hydrogen peroxide added to 100 g of DFC is hereinafter designated as the "percentage addition" of hydrogen peroxide, i.e. 2.5 mls added to 100 g DFC gives a "2.5%" hydrogen peroxide treatment—corresponding to the addition of 86 m moles per litre of hydrogen peroxide.

Samples of SCP obtained as described above were included in the diets of the following fowls:—

(a)   Broiler chicks (Ross I) fed diets containing 25% of the test sample from approximately 8 to 22 days of age.
(b)   Laying hens (Light White Hybrids over 40 weeks of age) fed diets containing 30% of the test sample for 6 days.

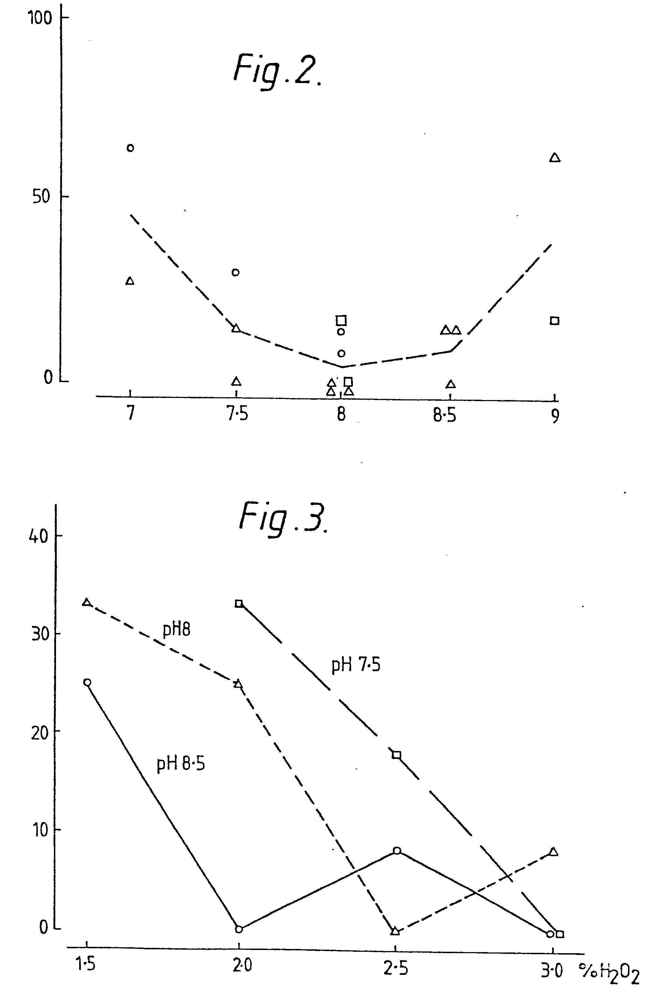

In each of a series of 6 trials SCP was treated with "2.5%" hydrogen peroxide at various pH values and was included in the diets of the above fowls (4 trials with chicks and 2 with laying hens). In each case a control diet containing untreated SCP was also used. The biological data generated from these trials are summarised in the graph shown in Figure 2. In this the ordinate is the percentage incidence of liver damage, relative to that occurring with a control diet including untreated SCP in the same trial (taken as 100%), using diets containing SCP treated with "2.5%" hydrogen peroxide. The abscissa is the pH of hydrogen peroxide treatment for the treated SCP. The different symbols on the graph represent the different trials from which the values plotted were obtained. The percentage of fowls exhibiting liver damage amongst those fed a control diet containing untreated SCP in each trial is given in Table 2.

TABLE 2

| Trial No. and symbol on Fig. 2 | % Incidence of liver damage in control | Type of fowl |
|---|---|---|
| ● BC 117 | 22.0% | chicks |
| ▲ APC003J | 32.7% | chicks |
| ○ APC016J | 27.0% | chicks |
| △ APC021J | 14.0% | chicks |
| □ L 32 | 30.0% | laying hens |
| ■ PL4 XXV | 27.8% | laying hens |

The percentages given in Table 2 were taken as the 100% values for the graph in Figure 2 in respect of the individual trials.

These data clearly indicate a pH optimum for such hydrogen peroxide treatment in the region of pH 8—8.5. The average percentage incidences of liver damage represented by the broken line in the graph in Figure 2 are:—

Untreated Control    —    25.6%
DFC/2.5% $H_2O_2$ . pH 7    —    11.7%
pH 7.5    —    3.6%

5

pH 8 — 1.4%
pH 8.5 — 2.3%
pH 9 — 10.2%

In a further trial APC024J carried out in the manner described above the level of hydrogen peroxide addition and the pH of treatment with hydrogen peroxide were both varied. The liver damage data resulting from this trial are summarised in the graph shown in Figure 3. In this graph the ordinate is the percentage liver damage incidence, relative to that occurring when an untreated control was used (taken as 100%), using feeds containing SCP treated with varying levels of hydrogen peroxide at varying pH values. The abscissa is the pH of hydrogen peroxide treatment. These data imply that an optimum treatment lies in the range "2%" $H_2O_2$ at pH 8 and "2.5%" $H_2O_2$ at pH 8.5.

**Claims**

1. A process for the production of single cell protein which comprises cultivating prokaryotic microorganisms and subsequently treating the cultivated microorganisms to produce a product comprising whole non-viable cells and/or ruptured cells wherein the microorganisms are treated with an oxidising agent at a pH between 7.0 and 9.0, the oxidising agent being added during cultivation and/or during the subsequent treatment and/or to the product after the subsequent treatment, the oxidising agent being added in an amount between 3 and 300 m moles per litre of a solids suspension containing the microorganisms when it is added during cultivation or during subsequent treatment, or, when added to the product, being added in an amount between 3 and 300 m moles per litre of the product moisture content.

2. A process according to claim 1 wherein the prokaryotic microorganism is a bacterium.

3. A process according to claim 2 wherein the bacterium is a strain of the species *Methylophilus methylotrophus*.

4. A process according to claim 3 wherein the bacterium is any one of strains NCIB Nos. 10508 to 10515 or 10592 to 10596.

5. A process according to any one of the preceding claims wherein, during production of the single cell protein, a concentrated suspension of microorganism cells is prepared and is subjected to drying means, the oxidising agent being added to the concentrated suspension before said suspension is subjected to drying means.

6. A process according to any one of claims 1 to 4 wherein the oxidising agent is added during subsequent treatment.

7. A process according to any one of the preceding claims wherein the microorganisms are treated with the oxidising agent at a temperature in the range 10° to 140°C.

8. A process according to claim 7 wherein treatment takes place at a temperature in the range 50° to 80°C.

9. A process according to any one of the preceding claims wherein the pH is in the range 7.5 to 8.5.

10. A process according to any one of the preceding claims wherein the oxidising agent is added in an amount in the range 30 to 175 m moles per litre of the solids suspension or per litre of the moisture content.

11. A process according to claim 10 wherein the oxidising agent is added in an amount in the range 70 to 150 m moles per litre.

12. A process according to any one of the preceding claims wherein the duration of the treatment with the oxidising agent is in the range 1 to 300 minutes.

13. A process according to claim 12 wherein the duration of the treatment is in the range 10 to 40 minutes.

14. A process according to any one of the preceding claims wherein the oxidising agent is hydrogen peroxide.

15. A feedstuff containing single cell protein produced by the process claimed in claim 1.

**Patentansprüche**

1. Verfahren zur Herstellung von Einzellerprotein, bei dem prokaryotische Mikroorganismen gezüchtet und die gezüchteten Mikroorganismen nachfolgend zur Herstellung eines Produkts, das ganze, nicht lebensfähige Zellen und/oder zerrissene Zellen enthält, behandelt werden, wobei die Mikroorganismen bei einem pH-Wert zwischen 7,0 und 9,0 mit einem Oxidationsmittel das während der Züchtung und/oder während der nachfolgenden Behandlung und/oder zu dem Produkt nach der nachfolgenden Behandlung zugegeben wird, behandelt werden, wobei das Oxidationsmittel im Fall seiner Zugabe während der Züchtung oder während der nachfolgenden Behandlung in einer Menge zwischen 3 und 300 mmol pro Liter einer die Mikroorganismen enthaltenden Feststoffsuspension oder im Fall seiner Zugabe zu dem Produkt in einer Menge zwischen 3 und 300 mmol pro Liter der in dem Produkt enthaltenen Feuchtigkeit zugegeben wird.

2. Verfahren nach Anspruch 1, bei dem der prokaryotische Mikroorganismus ein Bakterium ist.

3. Verfahren nach Anspruch 2, bei dem das Bakterium ein Stamm der Art *Methylophilus methylotrophus* ist.

4. Verfahren nach Anspruch 3, bei dem das Bakterium einer der Stämme mit den NCIB-Nummern 10508 bis 10515 oder 10592 bis 10596 ist.

5. Verfahren nach einem der vorhergehenden Ansprüche, bei dem während der Herstellung des Einzellerproteins eine eingedickte Suspension von Mikroorganismenzellen hergestellt und einer Trocknungsmaßnahme unterzogen wird, wobei das Oxidationsmittel zu der eingedickten Suspension zugegeben wird, bevor diese Suspension der Trockungsmaßnahme unterzogen wird.

6. Verfahren nach einem der Ansprüche 1 bis 4, bei dem das Oxidationsmittel während der nachfolgenden Behandlung zugegeben wird.

7. Verfahren nach einem der vorhergehenden Ansprüche, bei dem die Mikroorganismen bei einer Temperatur im Bereich von 10°C bis 140°C mit dem Oxidationsmittel behandelt werden.

8. Verfahren nach Anspruch 7, bei dem die Behandlung bei einer Temperatur im Bereich von 50°C bis 80°C stattfindet.

9. Verfahren nach einem der vorhergehenden Ansprüche, bei dem der pH-Wert in dem Bereich von 7,5 bis 8,5 liegt.

10. Verfahren nach einem der vorhergehenden Ansprüche, bei dem das Oxidationsmittel in einer Menge in dem Bereich von 30 bis 175 mmol pro Liter der Feststoffsuspension oder pro Liter der in dem Produkt enthaltenen Feuchtigkeit zugegeben wird.

11. Verfahren nach Anspruch 10, bei dem das Oxidationsmittel in einer Menge in dem Bereich von 70 bis 150 mmol pro Liter zugegeben wird.

12. Verfahren nach einem der vorhergehenden Ansprüche, bei dem die Dauer der Behandlung mit dem Oxidationsmittel in dem Bereich von 1 bis 300 min liegt.

13. Verfahren nach Anspruch 12, bei dem die Dauer der Behandlung in den Bereich von 10 bis 40 min liegt.

14. Verfahren nach einem der vorhergehenden Ansprüche, bei dem das Oxidationsmittel Wasserstoffperoxid ist.

15. Futtermittel, enthaltend durch das Verfahren nach Anspruch 1 hergestelltes Einzellerprotein.

## Revendications

1. Procédé pour la production de protéine monocellulaire consistant à cultiver des micro-organismes procaryotiques, puis à traiter les micro-organismes cultivés pour donner un produit comprenant des cellules entières non-viables et/ou des cellules brisées, caractérisé en ce que les micro-organismes sont traités par un agent oxydant à un pH compris entre 7,0 et 9,0, l'agent oxydant étant ajouté pendant la culture et/ou pendant le traitement ultérieure et/ou au produit après le traitement ultérieur, l'agent oxydant étant ajouté en une quantité comprise entre 3 et 300 mmoles par litre d'une suspension de solides contenant les micro-organismes quand il est ajouté pendant la culture ou pendant le traitement ultérieur, ou bien, quand il est ajouté au produit, étant ajouté en une quantité comprise entre 3 et 300 mmoles par litre de teneur en humidité du produit.

2. Procédé suivant la revendication 1, caractérisé en ce que le micro-organisme procaryotique est une bactérie.

3. Procédé suivant la revendication 2, caractérisé en ce que la bactérie est une souche de l'espèce Methylophilus methylotrophus.

4. Procédé suivant la revendication 3, caractérisé en ce que la bactérie est l'une quelconque des souches NCIB No. 10508 à 10515 ou 10592 à 10596.

5. Procédé suivant l'une quelconque des revendications précédentes, caractérisé en ce que, pendant la production de la protéine monocellulaire, on prépare une suspension concentrée de cellules de micro-organisme et on la soumet à des moyens de séchage, l'agent oxydant étant ajouté à la suspension concentrée avant que cette suspension ne soit soumise à l'action des moyens de séchage.

6. Procédé suivant l'une quelconque des revendications 1 à 4, caractérisé en ce que l'agent oxydant est ajouté pendant le traitement ultérieur.

7. Procédé suivant l'une quelconque des revendications précédentes, caractérisé en ce que les micro-organismes sont traités avec l'agent oxydant à une température comprise entre 10° et 140°C.

8. Procédé suivant la revendication 7, caractérisé en ce que le traitement a lieu à une température comprise entre 50° et 80°C.

9. Procédé suivant l'une quelconque des revendications précédentes, caractérisé en ce que le pH est compris dans une gamme allant de 7,5 à 8,5.

10. Procédé suivant l'une quelconque des revendications précédentes, caractérisé en ce que l'agent oxydant est ajouté en une quantité comprise dans une gamme allant de 30 à 175 mmoles par litre de suspension de solides ou par litre de teneur en humidité.

11. Procédé suivant la revendication 10, caractérisé en ce que l'agent oxydant est ajouté en une quantité comprise dans une gamme allant de 70 à 150 mmoles par litre.

12. Procédé suivant l'une quelconque des revendications précédentes, caractérisé en ce que la durée du traitement par l'agent oxydant est comprise dans une gamme allant de 1 à 300 minutes.

13. Procédé suivant la revendication 12, caractérisé en ce que la durée du traitement est comprise dans une gamme allant de 10 à 40 minutes.

14. Procédé suivant l'une quelconque des revendications précédentes, caractérisé en ce que l'agent oxydant est le peroxyde d'hydrogène.

15. Produit alimentaire contenant une protéine monocellulaire produite par le procédé suivant la revendication 1.

# Fig.1.

1

Fig.2.

Fig.3.

pH8

pH 7·5

pH 8·5

%H$_2$O$_2$